# EUROPÄISCHE PATENTANMELDUNG

(11) **EP 4 553 146 A2**
(43) Veröffentlichungstag der Anmeldung: **14.05.2025**
(21) Anmeldenummer: 25167260.6
(22) Anmeldetag: 03.03.2023
(51) Int. Cl.: C12M 3/06

(54) **VORRICHTUNG ZUM SCHÜTTELN VON PROBEN**

(30) Priorität: 04.03.2022 WO PCT/EP2022/055638
(62) Teilanmeldung aus: 23709657.3
(71) Anmelder: Infors AG, 4103 Bottmingen (CH)
(72) Erfinder: Hawrylenko, Alexander, 4103 Bottmingen (CH); Doppler, Johan, 68480 Lutter (FR)
(74) Vertreter: E. Blum & Co. AG

(57) **Zusammenfassung**

Die Erfindung betrifft eine Vorrichtung zum Schütteln von Proben umfassend einen Träger (62) und ein Antriebselement (66), das um eine Antriebsachse drehbar am Träger (62) gelagert und durch einen Antrieb (65) antreibbar ist. Weiterhin umfasst die Vorrichtung ein Tablar (61) eingerichtet zur Beladung mit den Proben (69), eine Tablarwelle (67), die mit dem Tablar (61) verbunden und exzentrisch an dem Antriebselement (66) gelagert ist, sowie ein an dem Antriebselement (66) befestigtes Gegengewicht (66a), das angepasst ist, eine im Betrieb der Vorrichtung mit einer definierten Beladung des Tablars (61) auftretende Unwucht auszugleichen. Ein Schwerpunkt des Gegengewichts (66a) befindet sich bezogen auf die Antriebsachse des Antriebselements (66) gegenüber der Tablarwelle (67). Der Schwerpunkt des Gegengewichts (66a) und ein Schwerpunkt des Tablars (61) samt Tablarwelle (67) und definierter Beladung befinden sich in derselben, orthogonal zur Antriebsachse verlaufenden Ebene. Bei Rotation des Antriebselements (66) um die Antriebsachse ist ein erstes Drehmoment, das durch das Tablar (61) samt Tablarwelle (67) und definierter Beladung auf die Antriebsachse ausgeübt wird, betragsgleich und entgegengesetzt gerichtet zu einem zweiten Drehmoment, das durch das Gegengewicht (66a) auf die Antriebsachse ausgeübt wird. Dadurch ist das Gegengewicht (66a) so angepasst, dass es sowohl eine statische als auch eine dynamische Unwucht auf die Antriebsachse ausgleicht. Entsprechend erreicht eine Schüttelfrequenz des Tablars (61) infolge des Antriebs (65) mindestens 1000 rpm, insbesondere mindestens 2000 rpm.

## Beschreibung

### Gebiet der Erfindung

Die Erfindung betrifft eine Vorrichtung zum Schütteln von Proben, insbesondere einen Laborschüttler, insbesondere für das Schütteln und/oder Durchmischen von Flüssigkeit enthaltenden Proben. Die Erfindung ist vorteilhaft für die Kultivierung von Bakterien und Zellen in flüssigen Nährmedien.

### Hintergrund

Schüttler werden verwendet, um Flüssigkeiten, z.B. Zellkulturen, Biotreibstoffe oder Blutproben, in Gefässen in einer Orbitalbewegung zu schütteln und/oder zu durchmischen. Häufig umfasst die geschüttelte Einheit ein Tablar, auf welchem sich die Gefässe, z.B. Erlenmeyerkolben, Reagenzgläser oder sonstige Ampullen, mit den Proben befinden. Für eine gute Durchmischung ist eine hohe Schüttelfrequenz wünschenswert. Gleichzeitig ermöglicht eine hohe Schüttelfrequenz einen schnellen Sauerstoff-Transfer von der Gasphase in die Flüssigphase, wodurch ein gutes Wachstum der Zellkulturen ermöglicht wird.

Gerade bei hohen Schüttelfrequenzen treten während des Schüttelvorgangs aber erhebliche Unwuchten an den rotierenden Bauteilen auf, z.B. an einer Antriebswelle und/oder an einem Antriebselement, an dem das Tablar gelagert ist. Eine solche Unwucht führt beim Schüttelvorgang zu Vibrationen des Schüttlers und dadurch zu Lärm sowie zu schädlichen Kräften auf Lager, mit denen die rotierenden Bauteile gelagert sind, was wiederum zu erhöhtem Verschleiss führt. Insofern limitiert eine Unwucht an einem rotierenden Bauteil in der Praxis die maximal erreichbare Schüttelfrequenz für einen Dauerbetrieb des Schüttlers. Dies hat zur Folge, dass die Kultivierung von Zellen in einem herkömmlichen Schüttler nur ein wesentlich langsameres Wachstum und die Produktion von deutlich weniger Biomasse ermöglicht als eine Kultivierung in einem Bioreaktor.

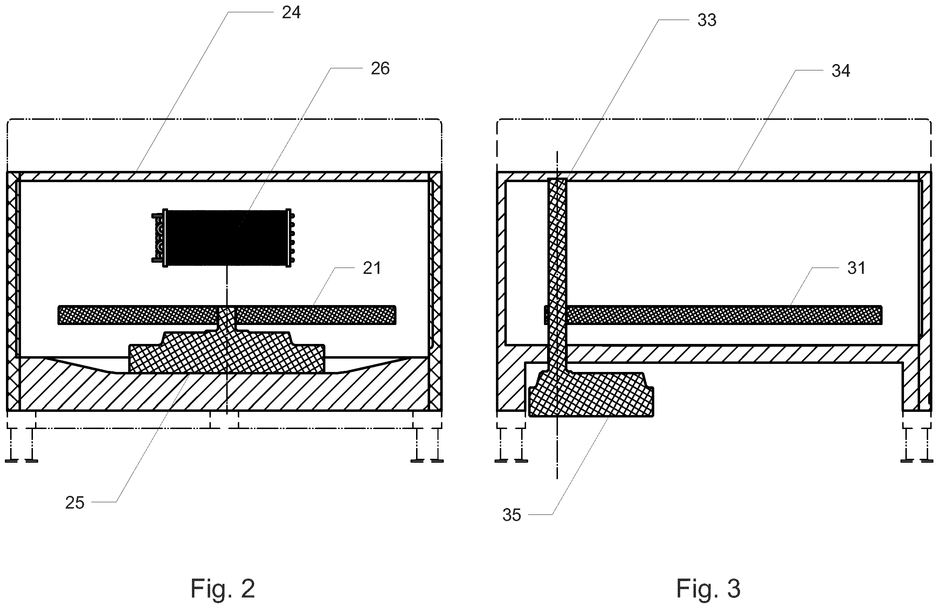

EP 3479894 A1 stellt einen Schüttler vor, bei dem das Tablar mit einer Tablarwelle exzentrisch in einer antreibbaren Hohlwelle gelagert ist. Zur Balancierung einer durch exzentrische Massenverteilung auftretenden Unwucht ist dort ein Gegengewicht an der Hohlwelle vorgesehen. Allerdings hat dieses Gegengewicht (Bezugszeichen 17 in der dortigen Fig. 2) den Nachteil, dass es eine Unwucht der Hohlwelle nur teilweise ausgleicht, da es insbesondere nur eine statische Unwucht ausgleicht.

Es stellt sich daher die Aufgabe, eine Vorrichtung zum Schütteln von Proben bereitzustellen, die eine bessere Durchmischung der Proben und/oder eine ausreichend gute Durchmischung von grösseren Proben ermöglicht. Alternativ kann die Aufgabe darin gesehen werden, eine Vorrichtung zum Schütteln von Proben bereitzustellen, die für einen Dauerbetrieb mit hoher Schüttelfrequenz über 1000 rpm, insbesondere über 1500 rpm, 2000 rpm oder 2500 rpm, z.B. bei 3 mm Durchmesser der Orbitalbewegung, geeignet ist.

### Darstellung der Erfindung

Diese Aufgabe wird gelöst von einer Vorrichtung zum Schütteln von Proben, einem sogenannten Schüttler, gemäss Anspruch 1. Unter einer Probe wird dabei insbesondere ein Stoff oder eine Stoffzusammensetzung verstanden, welche eine Flüssigkeit enthält, z.B. eine Zellkultur, einen Biotreibstoff oder eine Blutprobe. Die Probe ist üblicherweise in einem Gefäss, z.B. einem Reagenzglas oder einer Mikrotiterplatte, enthalten, das wiederum von einem Gefässhalter oder -ständer gehalten werden kann.

Die Vorrichtung umfasst
- einen Träger: Der Träger ist insbesondere eingerichtet zum Tragen oder Unterstützen eines Tablars.
- ein Antriebselement, das um eine Antriebsachse drehbar am Träger gelagert und durch einen Antrieb, z.B. von einem Motor, antreibbar ist: Das Antriebselement kann beispielsweise eine Hohlwelle oder eine Antriebsscheibe umfassen.
- ein Tablar eingerichtet zur Beladung mit den Proben: Insbesondere ist das Tablar ein flächiges Element, auf dem die Proben angebracht werden können. Vorteilhafterweise umfasst das Tablar Befestigungselemente zum Befestigen mindestens eines Gefässes mit einer Probe bzw. zum Befestigen mindestens eines Gefässhalters oder -ständers.
- eine Tablarwelle, die, insbesondere fest, mit dem Tablar verbunden und exzentrisch an dem Antriebselement gelagert ist: Vorteilhafterweise ist die Tablarwelle zentral an dem Tablar befestigt, insbesondere nahe eines Schwerpunkts des Tablars bzw. des Tablars mit einer definierten Beladung mit Proben. Als "nahe" ist insbesondere ein Bereich um den Schwerpunkt bis zu +/-10% von Länge und Breite des Tablars bezeichnet. Durch die zentrale Befestigung unterstützt die Tablarwelle (bei definierter Beladung) das Tablar nahe dem Schwerpunkt.

Als Exzentrizität (der Lagerung) der Tablarwelle an dem Antriebselement wird der Abstand zwischen einer Drehachse der Tablarwelle und der Antriebsachse des Antriebselements bezeichnet, welche insbesondere parallel zueinander verlaufen. Die Exzentrizität bestimmt eine Auslenkung des Tablars (und damit der Proben) beim Schütteln. Insbesondere kann die Exzentrizität der Lagerung der Tablarwelle an dem Antriebselement zwischen 0.5 und 3 mm betragen, insbesondere zwischen 1 und 2 mm. Dies führt zu einer Auslenkung des Tablars, welche für das Schütteln von Proben in kleineren Gefässen, z.B. Reagenzgläsern und Mikrotiterplatten, angepasst ist.

Die erreichbare Schüttelfrequenz des Tablars infolge des Antriebs beträgt mindestens 1000 rpm, insbesondere mindestens 1500 rpm, mindestens 2000 rpm oder mindestens 2500 rpm. So hohe Schüttelfrequenzen sind bis bisherigen Schüttlern, insbesondere infolge von Unwuchten, nicht erreichbar. Eine Schüttelfrequenz in diesem Bereich führt zu einer guten Durchmischung der Proben insbesondere in kleineren Gefässen, z.B. Reagenzgläsern und Mikrotiterplatten, insbesondere mit einem Volumen von bis zu 1 ml. Ausserdem lässt sich mit einer Schüttelfrequenz in diesem Bereich auch eine ausreichende Durchmischung von grösseren Proben, insbesondere mit einem Volumen von über 1 ml, z.B. 2 ml, erreichen, was mit einer geringeren Schüttelfrequenz nicht möglich ist. Mit der erfindungsgemässen Schüttelvorrichtung lassen sich also grössere Mengen an Zellen, z.B. in höheren Gefässen, kultivieren, was die Kultivierung erheblich effizienter macht.

Weiterhin umfasst die Vorrichtung ein an dem Antriebselement befestigtes Gegengewicht, das angepasst ist, eine im Betrieb der Vorrichtung mit einer definierten Beladung des Tablars auftretende Unwucht auszugleichen. Die definierte Beladung des Tablars mit Proben ist insbesondere charakterisiert durch eine Masse, z.B. einer Anzahl von Proben mal mittlere Masse der Proben plus Masse der Gefässe und etwaiger Gefässhalter, und eine Massenverteilung auf dem Tablar, z.B. eine Verteilung bzw. einen Schwerpunkt der Proben, Gefässe und etwaiger Gefässhalter auf dem Tablar. Für ein gutes Ausgleichen der Unwucht weichen die Masse und die Massenverteilung bzw. der Schwerpunkt der Beladung bevorzugt nur unwesentlich von den definierten Werten, also der definierten Beladung, ab. In Bezug auf die Masse bedeutet dies insbesondere eine Abweichung von höchstens +/-25%, in Bezug auf den Schwerpunkt insbesondere eine Abweichung von höchstens 3 cm.

Von einer Unwucht spricht man insbesondere bei einem rotierenden Körper, dessen Rotationsachse nicht einer seiner Hauptträgheitsachsen entspricht. Eine statische Unwucht entsteht insbesondere, wenn die Rotationsachse nicht durch den Schwerpunkt des Körpers verläuft. Eine dynamische Unwucht (auch Momentenunwucht genannt) entsteht, wenn die Rotationsachse nicht mit einer der Hauptträgheitsachsen des Körpers übereinstimmt, sondern im Schwerpunkt gegenüber den Hauptträgheitsachsen gekippt ist. Im allgemeinen Fall weist ein Körper mit beliebiger Drehachse sowohl eine statische als auch eine dynamische Unwucht auf. Die Unwucht kann eindeutig in einen statischen und einen dynamischen Anteil zerlegt werden. Der statische Anteil führt zu einer umlaufenden, resultierenden Kraft, der dynamische zu einem umlaufenden, resultierenden Drehmoment. Sowohl resultierende Kraft als auch resultierendes Drehmoment nehmen quadratisch mit der Drehzahl zu.

Erfindungsgemäss ist das Gegengewicht so angepasst, dass es sowohl eine statische als auch eine dynamische Unwucht auf die Antriebsachse ausgleicht. Dabei stellt das Gegengewicht vorteilhafterweise das einzige Gegengewicht an dem Antriebselement dar. Insbesondere befindet sich auf der dem Tablar gegenüberliegenden Seite des Antriebselements kein weiteres Gegengewicht. Dies ist ein Unterschied zu herkömmlichen Unwuchtausgleichen bei Schüttlern: Mit einem Gegengewicht wird herkömmlicherweise nur die statische Unwucht ausgeglichen, während für den Ausgleich der dynamischen Unwucht ein weiteres Gegengewicht benötigt würde.

Die Vorrichtung mit dem Gegengewicht, das sowohl eine statische als auch eine dynamische Unwucht auf die Antriebsachse ausgleicht, hat mehrere Vorteile: Einerseits lässt sich damit das resultierende Drehmoment auf die Antriebsachse und insbesondere auf deren Lagerung vermeiden. Dadurch entstehen gerade bei hohen Schüttelfrequenzen (von insbesondere 1000 rpm und mehr) weniger Lärmemissionen und weniger Verschleiss, sodass die Lebensdauer der Vorrichtung erhöht wird. Andererseits lässt sich die Vorrichtung mit dem (einzigen) Gegengewicht kompakt bauen, was insbesondere für Schüttler mit mehreren Tablaren übereinander von Vorteil ist. Ausserdem lassen sich hohe Schüttelfrequenzen, insbesondere von mehr als 1000, 1500, 2000 oder 2500 rpm, mit dem beschriebenen Gegengewicht überhaupt erst erreichen. Dies wiederum führt zu einer besseren Durchmischung der geschüttelten Proben sowie einem schnelleren Sauerstoff-Transfer von der Gasphase in die Flüssigphase, wodurch ein gutes Wachstum von Zellkulturen in den Proben ermöglicht wird.

### Gegengewicht

Zum Ausgleichen der statischen und dynamischen Unwucht befindet sich ein Schwerpunkt des Gegengewichts bezogen auf die Antriebsachse des Antriebselements gegenüber der Tablarwelle. Da über die Tablarwelle das Gewicht des Tablars samt Beladung exzentrisch auf das Antriebselement einwirkt, ist das Gegengewicht so an dem Antriebselement angebracht, dass der Schwerpunkt des Gegengewichts in Bezug auf die Antriebsachse der Tablarwelle gegenüber liegt. Insbesondere läuft der Schwerpunkt des Gegengewichts wie auch die Tablarwelle bei einer Rotation des Antriebselements um die Antriebsachse um. Bei geeigneter Wahl von Masse und Schwerpunkt des Gegengewichts kann dadurch die statische Unwucht ausgeglichen werden.

Ausserdem befinden sich der Schwerpunkt des Gegengewichts und ein Schwerpunkt des Tablars samt Tablarwelle und definierter Beladung in derselben, orthogonal zur Antriebsachse verlaufenden Ebene. Dadurch wird das Auftreten einer dynamischen Unwucht vermieden.

Weiterhin ist bei Rotation des Antriebselements um die Antriebsachse ein erstes Drehmoment, das durch das Tablar samt Tablarwelle und definierter Beladung auf die Antriebsachse ausgeübt wird, betragsgleich und entgegengesetzt gerichtet zu einem zweiten Drehmoment, das durch das Gegengewicht auf die Antriebsachse ausgeübt wird. So können wiederum eine statische und eine dynamische Unwucht auf das Antriebselement vermieden werden.

In einer Ausführungsform weist das Tablar im Bereich der Tablarwelle eine Ausstülpung auf, innerhalb derer sich das Gegengewicht zumindest teilweise befindet. Insbesondere befindet sich der Schwerpunkt des Gegengewichts innerhalb der Ausstülpung. Dies hat den Vorteil, dass das Gegengewicht wie auch die weiteren Bauteile, z.B. Tablarwelle und Antriebselement, durch das Tablar vor Verschmutzung, z.B. übergelaufene oder verspritzte Proben, geschützt sind. Dadurch wird ein Reinigen der Vorrichtung nach Verschmutzung vereinfacht. Gleichzeitig kann durch die Ausstülpung und spezifische Anordnung des Gegengewichts erreicht werden, dass der Schwerpunkt des Gegengewichts und der Schwerpunkt von Tablar samt Tablarwelle und definierter Beladung (wie oben beschrieben) in derselben Ebene liegen.

Vorteilhafterweise weist das Gegengewicht eine Masse zwischen 0.1 und 1 kg, insbesondere zwischen 0.5 und 0.9 kg, auf. Ausserdem kann das Gegengewicht aus Metall, insbesondere aus Gusseisen oder rostfreiem Stahl, hergestellt sein. Ein solches Gegengewicht lässt sich aufgrund seiner hohen Dichte kompakt dimensionieren und platzsparend in der Ausstülpung anbringen. Ausserdem braucht ein solches Gegengewicht nur um einige Zentimeter, insbesondere zwischen 1 und 5 cm, z.B. zwischen 2 und 3 cm, beabstandet zur Antriebsachse angebracht werden, um z.B. eine Unwucht bei den oben genannten vorteilhaften Exzentritäten zwischen 0.5 und 3 mm, insbesondere zwischen 1 und 2 mm, und einer Beladung des Tablars von z.B. 15, 20 oder 25 kg auszugleichen.

In einer Ausführungsform weist das Tablar eine rechteckige Form auf. Dies erlaubt eine platzsparende Beladung des Tablars mit typischen Gefässen und Gefässhaltern. Insbesondere kann das Tablar eine Länge zwischen 50 und 100 cm und/oder eine Breite zwischen 30 und 70 cm aufweisen. Ein solches Tablar eignet sich sowohl für einen Tischschüttler, der als eigenständiges Gerät z.B. auf einem Labortisch platziert wird, als auch für einen Mehrfachschüttler mit mehreren Tablaren übereinandern, die z.B. von einem Gehäuse umschlossen sind.

### Rotationssicherung des Tablars

Vorteilhafterweise ist das Tablar gegen Rotation gegenüber dem Träger gesichert, um die erwünschte Orbitalbewegung zu erzeugen. Ansonsten würde das Tablar bei Rotation des Antriebselements ebenfalls um die Antriebsachse rotieren. Gewünscht ist für den Schüttelvorgang aber eine Orbitalbewegung, also eine Translation des Tablars samt Beladung auf einer kreisförmigen Bahn, die insbesondere durch die Exzentrizität der Lagerung der Tablarwelle am Antriebselement gegeben ist. Eine solche Translation kann auf verschiedene Arten erreicht werden.

In einer Ausführungsform weist das Antriebselement eine erste Riemenscheibe zum Antreiben über einen Riemen auf. Ausserdem umfasst die Vorrichtung ein zweites Antriebselement und eine zweite Tablarwelle, die mit dem Tablar verbunden und exzentrisch an dem zweiten Antriebselement gelagert ist. Auch an dem zweiten Antriebselement ist ein Gegengewicht zum Ausgleichen der Unwucht befestigt wie oben beschrieben. Insbesondere befindet sich ein Schwerpunkt des zweiten Gegengewichts bezogen auf die zweite Antriebsachse des zweiten Antriebselements gegenüber der zweiten Tablarwelle. Ausserdem befinden sich der Schwerpunkt des zweiten Gegengewichts und der Schwerpunkt des Tablars samt zweiter Tablarwelle und definierter Beladung in derselben, orthogonal zur zweiten Antriebsachse verlaufenden Ebene. Weiterhin ist bei Rotation des zweiten Antriebselements um die zweite Antriebsachse ein drittes Drehmoment, das durch das Tablar samt zweiter Tablarwelle und definierter Beladung auf die zweite Antriebsachse ausgeübt wird, betragsgleich und entgegengesetzt gerichtet ist zu einem vierten Drehmoment, das durch das zweite Gegengewicht auf die zweite Antriebsachse ausgeübt wird. Vorteilhafterweise umfasst das zweite Antriebselement eine zweite Riemenscheibe, welche über einen Zahnriemen mit der ersten Riemenscheibe verbunden ist und so angetrieben wird. Vorteilhafterweise ist das Tablar an dem zweiten Antriebselement mit der gleichen Exzentrizität gelagert ist wie an dem Antriebselement samt erster Riemenscheibe, sodass das Tablar durch den synchronen Antrieb von erster und zweiter Riemenscheibe gegen Rotation gesichert wird.

In einer besonders vorteilhaften Ausführungsform mit erster und zweiter Riemenscheibe, erster und zweiter Tablarwelle und erstem und zweitem Antriebselement umfasst das Tablar einen ersten Tablarteil und einen zweiten Tablarteil. Die erste und zweite Tablarwelle sind, insbesondere fest, mit dem ersten bzw. zweiten Tablarteil verbunden und exzentrisch an dem ersten bzw. zweiten Antriebselement gelagert. Zur Verhinderung einer Rotation des Tablars sind der erste und der zweite Tablarteil durch eine Linearführung miteinander verbunden. Eine solche Linearführung ist insbesondere dazu eingerichtet, dass sich der erste und der zweite Tablarteil zwar entlang einer Achse der Linearführung gegeneinander verschieben, aber nicht gegeneinander verdrehen können. In anderen Worten beschränkt die Linearführung alle Freiheitsgrade der Tablarteile zueinander mit Ausnahme eines Translationsfreiheitsgrads entlang der Achse der Linearführung. Die Ausführungsform mit zwei Tablarteilen, die mit einer Linearführung verbunden sind, hat den Vorteil, dass schädliche Kräfte auf die Lagerung der Tablarwellen, die z.B. durch eine Wärmeausdehnung eines einstückigen Tablars bewirkt werden, verhindert werden. Damit lassen sich wiederum höhere Schüttelfrequenzen, eine längere Betriebsdauer sowie ein ruhigerer Lauf des Schüttlers erzielen.

Alternativ kann eine Rotation des Tablars auch dadurch verhindert werden, dass die Vorrichtung zusätzlich flexible Elemente und/oder Gelenkelemente umfasst, die am Träger oder an einem mit dem Träger verbundenen Gehäuse angebracht sind und eingerichtet sind, das Tablar zu führen. Vorteilhafterweise greifen die flexiblen Elemente und/oder Gelenkelemente in einem Randbereich des Tablars an.

### Gehäuse

In einer Ausführungsform umfasst die Vorrichtung zusätzlich ein öffenbares Gehäuse, wobei sich das Tablar, die Tablarwelle, das Antriebselement und der Träger in einem Innenraum innerhalb des Gehäuses befinden. Das Gehäuse kann einerseits dem Abschluss von Tablar und Proben von einer Umgebung der Vorrichtung dienen, z.B. um Verschmutzung der Umgebung durch Verspritzen von Flüssigkeit zu vermeiden. Andererseits kann das Gehäuse zur Klimatisierung des Innenraums eingerichtet sein. Dazu kann die Vorrichtung ein Klimasteuerelement umfassen, das eingerichtet ist zum Steuern von Temperatur und/oder Feuchtigkeit im Innenraum des Gehäuses. Zu diesem Zweck und insbesondere um ideale Umgebungsbedingungen für die Proben zu schaffen, kann das Klimasteuerelement z.B. einen Heizer, einen Kühler, einen Befeuchter und/oder einen Entfeuchter umfassen, der an dem Gehäuse angebracht sein kann. Zum öffenbaren Verschliessen des Gehäuses und damit zum Aufrechterhalten der idealen Umgebungsbedingungen umfasst das Gehäuse vorteilhafterweise eine Tür, z.B. eine schwenkbare Tür, insbesondere an einer Vorderseite des Gehäuses.

### Mehrere Tablare

In einer Ausführungsform umfasst die Vorrichtung mindestens ein weiteres Tablar samt weiterer Tablarwelle, weiterem Antriebselement, weiterem Gegengewicht und weiterem Träger im Innenraum des Gehäuses. Insbesondere kann die Vorrichtung mindestens fünf weitere Tablare, samt weiteren Tablarwellen, weiteren Antriebselementen, weiteren Gegengewichten und weiteren Trägern im Innenraum des Gehäuses umfassen. Dadurch wird die Kapazität der Vorrichtung, also insbesondere eine Anzahl gleichzeitig schüttelbarer Proben, erhöht. Vorteilhafterweise sind das Antriebselement sowie das mindestens eine weitere Antriebselement bzw. die mindestens fünf weiteren Antriebselemente über eine Hauptantriebswelle antreibbar. Durch den gemeinsamen Antrieb über die Hauptantriebswelle wird eine kompakte Bauweise mit einfacher Wartbarkeit erreicht.

Um eine Unwucht im Falle mehrerer Tablare zu vermeiden, ist es von Vorteil, dass eine Winkelposition einer Lagerung der weiteren Tablarwelle an dem weiteren Antriebselement abweicht von einer Winkelposition der Lagerung der Tablarwelle an dem Antriebselement. Ansonsten könnte, besonders bei schwerer Beladung der Tablare, eine Unwucht auf die Hauptantriebswelle wirken und die gesamte Vorrichtung zu Vibrationen anregen.

Dies kann insbesondere vermieden werden, indem sich die Winkelposition der Lagerungen der verschiedenen Tablarwellen zueinander um 360°/N unterscheiden, wobei N die Anzahl der Tablare in der Vorrichtung ist. Dadurch wird eine Unwucht auf die Hauptantriebswelle ausgeglichen.

### Antrieb

In einer Ausführungsform umfasst der Antrieb einen Motor, z.B. einen Elektromotor, zum Antreiben des Antriebselements und, wenn vorhanden, des mindestens einen weiteren Antriebselements. Der Motor kann insbesondere über ein Getriebe an die Hauptantriebswelle gekoppelt sein.

Vorteilhafterweise ist der Motor ausserhalb des Gehäuses angebracht, wenn ein solches vorhanden ist. Die Anbringung des Motors ausserhalb des Gehäuses hat den Vorteil, dass Wärme, welche beim Betrieb des Motors entsteht, nicht in den Innenraum des Gehäuses eingebracht wird. Für viele Anwendungen bzw. Proben ist eine Steuerung von Temperatur und/oder Feuchte, z.B. durch eine Klimasteuerung wie oben beschrieben, wünschenswert. Dabei wird insbesondere die Feuchtigkeit nahe des Taupunkts gehalten, insbesondere bei einer relativen Feuchte zwischen 80% und 100%. Wenn der Innenraum nun aber gleichzeitig gekühlt werden muss, z.B. wegen des Eintrags von Wärme durch einen Motor im Innenraum, erreicht die Feuchte lokal an der Klimasteuerung bzw. am Kühler 100% und kondensiert aus. Ein Kondensieren ist wiederum unerwünscht, weil dadurch eine unkontrollierte Vermehrung von Fremdkeimen auftreten kann, was schädlich für die Proben sein kann. Insbesondere soll der Innenraum des Gehäuses thermisch von dem Motor entkoppelt sein. Dieses Problem wird durch einen ausserhalb des Gehäuses angebrachten Motor gelöst.

Insbesondere kann der Motor an einer Unterseite des Gehäuses angebracht sein. Dies setzt einen Schwerpunkt der Vorrichtung herunter und erhöht somit die Standfestigkeit der Vorrichtung, insbesondere bei hohen Schüttelfrequenzen. Dabei ist die Hauptantriebswelle vorteilhafterweise durch eine Öffnung in der Unterseite des Gehäuses hindurchgeführt.

Es wird darauf hingewiesen, dass die verschiedenen Ausführungsformen, wo technisch sinnvoll, auch kombiniert werden können, wodurch synergistische Effekte und weitere Vorteile erreicht werden können.

### Kurze Beschreibung der Zeichnungen

Weitere Ausgestaltungen, Vorteile und Anwendungen der Erfindung ergeben sich aus den abhängigen Ansprüchen und aus der nun folgenden Beschreibung anhand der Figuren. Dabei zeigen:
Fig. 1a eine perspektivische Ansicht einer Vorrichtung zum Schütteln von Proben gemäss einer Ausführungsform der Erfindung; Fig. 1b eine Draufsicht von oben auf die Vorrichtung von Fig. 1a;
Fig. 2 einen schematischen Schnitt durch eine Vorrichtung zum Schütteln von Proben mit innenliegendem Motor gemäss dem Stand der Technik;
Fig. 3 einen schematischen Schnitt durch eine Vorrichtung zum Schütteln von Proben mit aussenliegendem Motor gemäss einer Ausführungsform der Erfindung;
Fig. 4 eine schematische Seitenansicht einer Ausführungsform der erfindungsgemässen Vorrichtung mit mehreren Tablaren;
Fig. 5a eine Schemazeichnung einer Vorrichtung zum Schütteln von Proben gemäss dem Stand der Technik, bei der eine statische Unwucht ausgeglichen wird; Fig. 5b eine Schemazeichnung einer Vorrichtung, bei der sowohl eine statische als auch eine dynamische Unwucht ausgeglichen wird;
Fig. 6a einen schematischen Schnitt vertikal durch eine Vorrichtung zum Schütteln von Proben gemäss einer Ausführungsform; Fig. 6b eine Detailansicht des Bereichs C von Fig. 6a.
Fig. 7 einen horizontalen Schnitt bzw. eine Draufsicht von oben auf ein Tablar und ein Gegengewicht gemäss einer Ausführungsform der Erfindung;
Figs. 8a, 8b und 8c Schemazeichnungen einer flüssigen Probe in einem Gefäss bei anwachsender Schüttelfrequenzen;
Fig. 9 einen schematischen Schnitt durch ein Lager, mit dem das Tablar gemäss einer Ausführungsform an der Hauptantriebswelle gelagert ist;
Fig. 10a eine perspektivische Ansicht eines schwenkbaren Tablars mit einer Verrastung, welche in Fig. 10b vergrössert dargestellt ist, gemäss einer Ausführungsform;
Figs. 11a, 11b und 11c eine Ausführungsform mit geteiltem Tablar in einer perspektivischen Ansicht (Fig. 11a), einem schematischen horizontalen Schnitt (Fig. 11b) und einem schematischen vertikalen Schnitt (Fig. 11c).

### Wege zur Ausführung der Erfindung

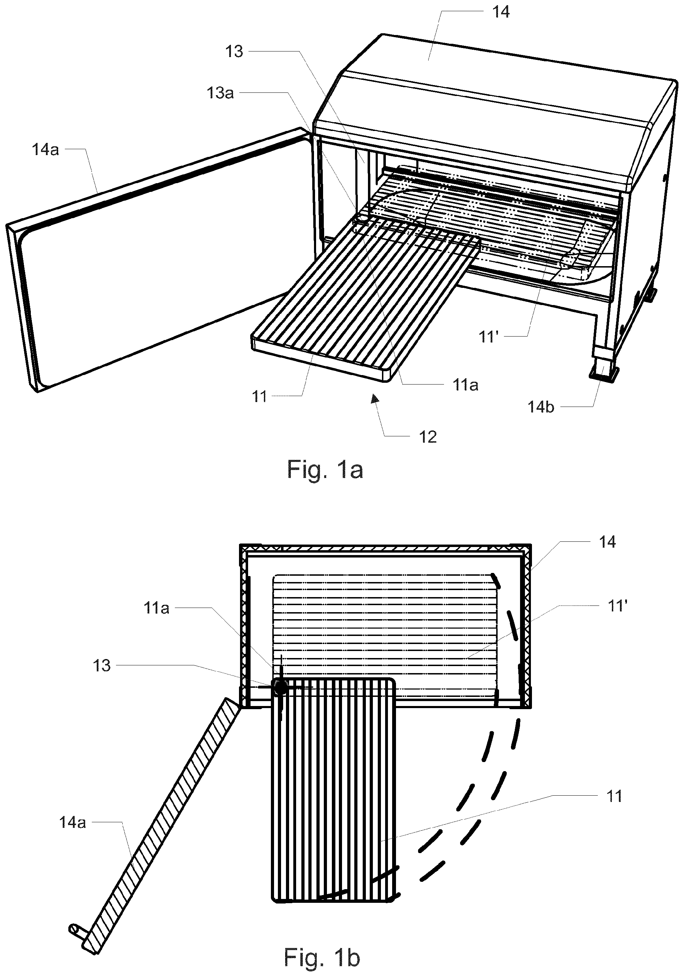

Figs. 1a und 1b zeigen eine Vorrichtung zum Schütteln von Proben, einen sogenannten Schüttler, gemäss einer Ausführungsform. Dabei ist Fig. 1a eine perspektivische Ansicht der Vorrichtung, während Fig. 1b eine Draufsicht von oben wiedergibt. Die Vorrichtung umfasst ein Tablar 11, das dazu eingerichtet ist, mit Proben beladen zu werden. Das Tablar 11 ist auf einem Träger 12 (in Figs. 1a und 1b nicht sichtbar) gelagert und wird von diesem gestützt. Der Träger 12 und mit ihm das Tablar 11 sind um eine Hauptantriebswelle 13 schwenkbar. Zu diesem Zweck ist der Träger 12 über ein Lager 13a, z.B. ein Kugellager, mit der Hauptantriebswelle 13 verbunden. Ausserdem weist das Tablar 11 an seinem Randbereich eine Öffnung 11a auf, durch welche die Hauptantriebswelle 13 hindurchtritt. Die Öffnung 11a ist dabei abhängig von der Exzentrizität der Lagerung des Tablars, insbesondere von der Auslenkung der Schüttelbewegung, grösser als ein Durchmesser der Hauptantriebswelle 13.

Das Tablar 11 umfasst mit Vorteil zumindest an seiner Oberseite, also der den Proben zugewandten Seite, eine leicht zu reinigende Oberfläche, z.B. aus Metall. Dies ermöglicht einen sterilen Betrieb der Vorrichtung. Weiterhin kann das Tablar 11 eine Standardgrösse von 850 mm x 470 mm aufweisen.

Das Tablar 11 im eingeklappten Zustand 11' sowie zumindest ein Teil der Hauptantriebswelle 13 können von einem Gehäuse 14 umschlossen sein, das zum Öffnen und Verschliessen eine Tür 14a umfasst. Das Gehäuse 14 erfüllt im Allgemeinen mehrere Funktionen: Zum einen bildet es einen ortsfesten Rahmen, der, z.B. über Füsse 14b, auf einem Tisch, in einem Labor oder allgemein auf einer Unterlage platziert werden kann. Die Schüttelbewegung des Tablars geschieht relativ zu diesem ortsfesten Rahmen. Zum zweiten bietet das Gehäuse einen Schutz der Umgebung der Vorrichtung, z.B. vor Verspritzen oder Überlaufen von Proben oder vor Dämpfen, was insbesondere bei schädlichen Proben oder in einem sterilen Labor wünschenswert ist. Zum dritten können in einem Innenraum des Gehäuses kontrollierte Bedingungen, z.B. in Bezug auf Temperatur und/oder Feuchtigkeit, eingestellt werden, wie es für viele Proben vorteilhaft ist. Zu diesem Zweck kann die Vorrichtung eine Klimasteuerung für den Innenraum umfassen (wie oben beschrieben, in Figs. 1a und 1b nicht gezeigt).

In Figs. 1a und 1b ist das Tablar in zwei Positionen gezeigt: einerseits (mit 11 bezeichnet) aus dem Gehäuse 14 herausgeschwenkt, andererseits (gestrichelt, mit 11' bezeichnet) im Gehäuse eingeschwenkt in betriebsbereitem Zustand. In der Figur beträgt der Winkel zwischen den beiden Positionen 90°. Im Allgemeinen ist aber bereits ein Winkel von mindestens 45° vorteilhaft, da er die Zugänglichkeit des Tablars 11 und des Innenraums des Gehäuses 14 verbessert.

Weiterhin ist in Figs. 1a und 1b erkennbar, dass eine Anbringung der Hauptantriebswelle 13 im Randbereich oder gar Eckbereichs des Tablars, insbesondere weniger als 20% einer Länge und/oder Breite des Tablars von einem Rand des Tablars entfernt, vorteilhaft ist für die Schwenkbarkeit und Zugänglichkeit des Tablars. Alternativ kann der gleiche Vorteil dadurch erreicht werden, dass die Hauptantriebswelle 13 ausserhalb des Tablars 11 nahe dessen Randbereich verläuft, insbesondere weniger als 20% der Länge oder Breite des Tablars vom Rand des Tablars entfernt (nicht gezeigt in Figs. 1a und 1b). In diesem Fall ist die Öffnung 11a im Tablar 11 überflüssig und der Träger 12 ragt in horizontaler Richtung über das Tablar 11 hinaus. Im Allgemeinen verbessert die Schwenkbarkeit des Tablars aus dem Gehäuse heraus die Zugänglichkeit der Proben. Insbesondere ermöglicht ein schwenkbares Tablar eine Automatisierung des Befüll- und Entnahmeprozesses der Proben, da z.B. ein Roboterarm die Vorrichtung einfacher computergesteuert bedienen kann.

Fig. 2 zeigt einen schematischen Schnitt durch einen Schüttler gemäss dem Stand der Technik. Ein Tablar 21 wird von einem Motor 25 angetrieben, welcher sich innerhalb eines Gehäuses 24 befindet. Die Anordnung des Motors 25 im Innenraum des Gehäuses 24 hat den Nachteil, dass Wärme, die vom Motor 25 generiert wird, direkt den Innenraum und damit darin befindliche Proben erwärmt. Für einige Proben ist es allerdings erforderlich, den Innenraum zu klimatisieren, insbesondere Temperatur und/oder Feuchtigkeit zu steuern, z.B. über eine Klimasteuerung 26. Wie oben beschrieben, kann dies zum Kondensieren von Feuchtigkeit im Innenraum, insbesondere an der Klimasteuerung 26 bzw. dem Kühler, führen, was wiederum die Proben beschädigen kann. Ein innenliegender Motor 25 trägt zu diesem Problem bei, da dem Innenraum die durch den Motor 25 generierte Wärme durch die Klimasteuerung 26 wieder entzogen werden muss.

Fig. 3 illustriert mit einem schematischen Schnitt durch einen Schüttler einen weiteren Aspekt der Erfindung. Im Gegensatz zu Fig. 2 (Stand der Technik) ist das Tablar 31 hier über eine Hauptantriebswelle 33 von einem Motor 35 antreibbar, welcher ausserhalb des Gehäuses 34 angebracht ist. Die Hauptantriebswelle 33 verläuft orthogonal zum Tablar 31 und zum grösseren Teil innerhalb des Gehäuses 34, während ein kleinerer Teil der Hauptantriebswelle 33 ausserhalb des Gehäuses verläuft. Die Anbringung des Motors 35 unterhalb des Gehäuses 34 ist im Hinblick auf einen tiefen Schwerpunkt der Vorrichtung vorteilhaft.

Ein ausserhalb des Gehäuses angebrachter Motor, insbesondere wie in Fig. 3, hat allgemein den Vorteil, dass die vom Motor erzeugte Wärme nicht in den Innenraum des Gehäuses eingebracht wird und diesen damit nicht erwärmt. Dadurch ist weniger Kühlleistung erforderlich, um den Innenraum auf einer konstanten Temperatur zu halten. So kondensiert im Innenraum, z.B. lokal an der Klimasteuerung bzw. dem Kühler, auch weniger Feuchtigkeit aus, welche für die Proben schädlich sein könnte. Dies erleichtert das Schaffen kontrollierter Umgebungsbedingungen im Innenraum, insbesondere einer konstanten Temperatur und einer hohen Feuchtigkeit, z.B. zwischen 80% und 100% relativer Feuchte ohne Kondensat.

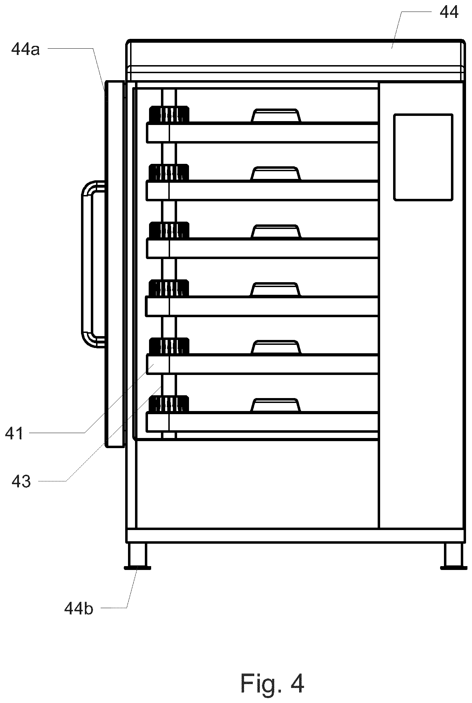

Fig. 4 illustriert einen Schüttler mit mehreren, insbesondere sechs, Tablaren 41, die in einem Gehäuse 44 mit Tür 44a von einer einzigen Hauptantriebswelle 43 antreibbar sind. So sind alle Tablare 41 von einem Motor (nicht gezeigt in Fig. 4) antreibbar, der wiederum ausserhalb des möglicherweise klimatisierten Innenraums des Gehäuses 44 angebracht sein kann, wie oben beschrieben. Vorteilhafterweise verläuft die Hauptantriebswelle 43 für eine optimale Schwenkbarkeit der Tablare 41 wiederum im Randbereich, insbesondere im Eckbereich, durch die Tablare 41 hindurch.

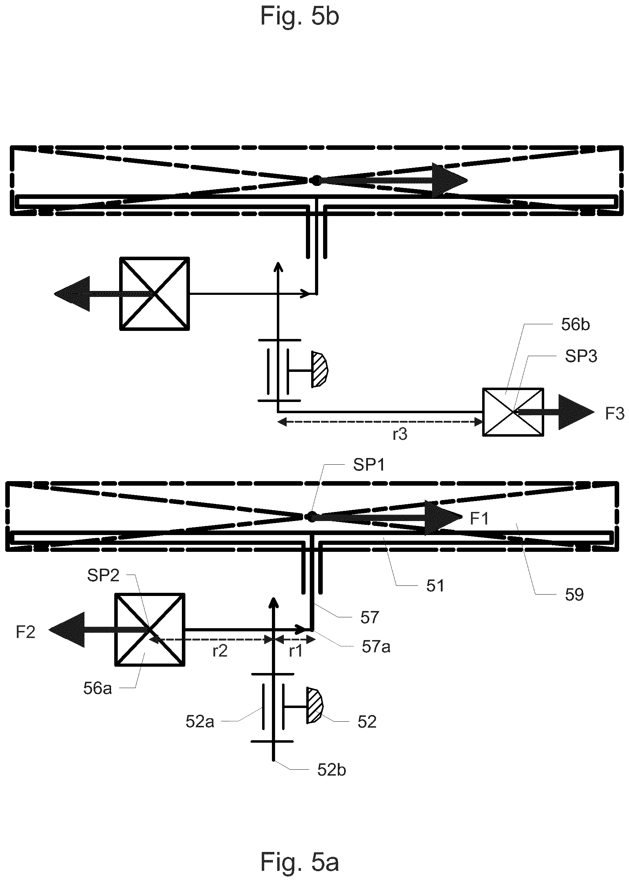

Fig. 5a zeigt anhand einer Prinzipskizze, wie eine statische Unwucht bei einem herkömmlichen Schüttler (wie im Abschnitt "Hintergrund" oben genannt) ausgeglichen werden kann. Ein Tablar 51 mit einer Beladung 59, umfassend z.B. Proben, Gefässe und Gefässhalter, ist über eine Tablarwelle 57 exzentrisch an einem Antriebselement, z.B. einer Hohlwelle, gelagert. Die Lagerung 57a der Tablarwelle 57 am Antriebselement liegt nicht auf der Antriebsachse 52b, um die das Antriebselement aufgrund seiner Lagerung 52a an einem Träger 52 rotiert. Vielmehr ist die Lagerung 57a aufgrund der Exzentrizität um den ersten Radius r1 von der Antriebsachse 52b beabstandet. Die Masse von Tablar 51, Beladung 59 und Tablarwelle 57 stellt daher eine Unwuchtmasse u1 dar, die zur Unwucht U1 = u1*r1 führt.

Gemäss dem Stand der Technik wird zum Ausgleichen der statischen Unwucht U1 ein Gegengewicht 56a so an dem Antriebselement angebracht, dass der Schwerpunkt SP2 des Gegengewichts 56a dem Schwerpunkt SP1 der Unwuchtmasse u1 in Bezug auf die Antriebsachse 52b gegenüber liegt. Dabei wird das Gegengewicht 56a z.B. in Richtung der Antriebsachse 52b zwischen dem Schwerpunkt SP1 und der Lagerung 52a des Antriebselements angebracht. Wenn die Unwucht U2 = u2*r2 des Gegengewichts 56a, mit Masse u2 des Gegengewichts und Abstand r2 des Schwerpunkts SP2 von der Antriebsachse 52b, gleich gross ist wie die Unwucht U1 = U2, wird die statische Unwucht gerade ausgeglichen. Mit anderen Worten würde sich das Antriebselement bei waagerechter Antriebsachse 52b aus einer beliebigen Anfangsstellung nicht ohne Einwirkung einer äusseren Kraft drehen.

Die Anordnung mit Gegengewicht 56a gemäss Fig. 5a weist aber weiterhin eine dynamische Unwucht auf, da die Unwuchten U1 und U2 in Richtung der Antriebsachse 52b um den Achsabstand l zueinander versetzt sind. Bei einer Rotation des Antriebselements (samt Tablar 51, Beladung 59, Tablarwelle 57 und Gegengewicht 56a) wirken Kräfte F1 = U1*ω² und F2 = U2*ω², siehe die dicken Pfeile in Fig. 5a, wobei ω die Kreisfrequenz ist. Mit U = U1 = U2 und entgegengesetzten Richtungen der Kräfte F1 und F2, F = F1 = -F2, bewirken dies Kräfte ein Drehmoment M = F1*l/2 - F2*l/2 = l*F = l*U*ω² senkrecht zur Antriebsachse 52b, das mit der Antriebsachse rotiert. Damit ergibt sich bei idealem Ausgleich der statischen Unwucht ein Deviationsmoment D = u*l*r = U*l.

Fig. 5b zeigt eine Prinzipskizze, wie durch ein zusätzliches Gegengewicht 56b auch die dynamische Unwucht ausgeglichen werden kann. Zu diesem Zweck ist das zusätzliche Gegengewicht 56b in Richtung der Antriebsachse 52b auf der anderen Seite der Lagerung 52a angebracht wie das Tablar 51 samt Beladung 59 und Tablarwelle 57 sowie das Gegengewicht 56a. Ausserdem ist das zusätzliche Gegengewicht 56b mit Masse u3, Schwerpunkt SP3 und Achsabstand r3 des Schwerpunkts SP3 in Bezug auf die Antriebsachse 52b auf der gleichen Seite angebracht wie der Schwerpunkt SP1, also auf der gegenüberliegenden Seite zu Schwerpunkt SP2. Dadurch wirkt bei einer Rotation um die Antriebsachse analog zu oben zusätzlich eine Kraft F3.

Auf diese Weise kann bei geeigneter Wahl von u3 und r3 zusätzlich zur statischen auch die dynamische Unwucht auf die Antriebsachse 52b ausgeglichen werden. Damit werden schädliche Kräfte und ein Verschleiss des Lagers 52a vermieden. Nachteilig an der Anordnung gemäss Fig. 5b ist aber die Notwendigkeit, zwei Gegengewichte 56a und 56b vorzusehen, was eine platzsparende Konstruktion verhindert.

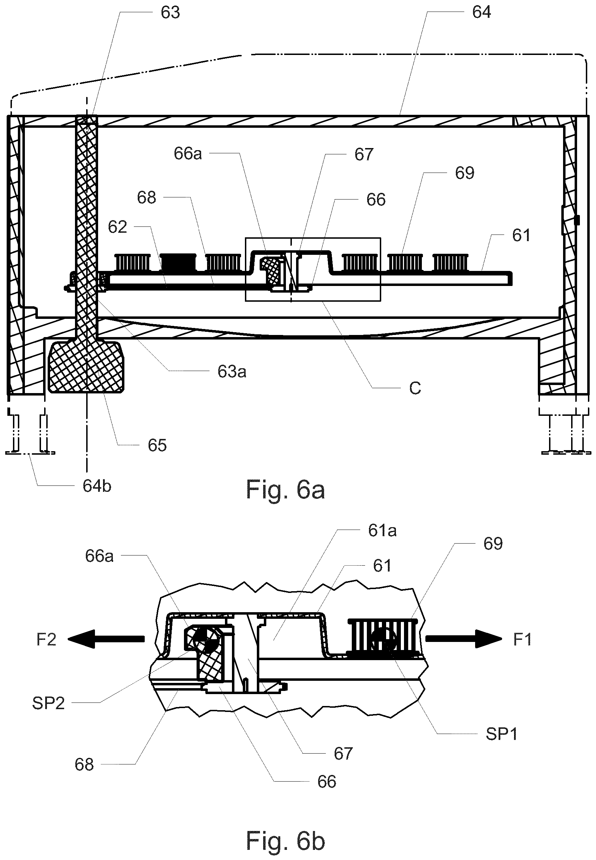

Ein Aspekt der vorliegenden Erfindung bezieht sich daher auf eine platzsparende Anordnung eines Gegengewichts, die sowohl die statische als auch die dynamische Unwucht ausgleicht. Eine solche Anordnung ist unten in Bezug auf Fig. 6b beschrieben.

Figs. 6a und 6b fokussieren auf den mechanischen Aspekt, wie ein Tablar 61 über einen Träger 62 mit einem Lager 63a an der Hauptantriebswelle 63 schwenkbar befestigt ist (Fig. 6a), sowie Details zum Antrieb des Tablars 61 über ein Antriebselement 66 mit Gegengewicht 66a (Fig. 6b). Prinzipiell sind die beschriebenen Mechanismen auch auf mehrere Tablare anwendbar, z.B. auf den Schüttler nach Fig. 4.

Das Tablar 61 ist dazu eingerichtet, mit einer oder mehreren Proben 69, z.B. in Mikrotiterplatten, beladen zu werden, die geschüttelt werden sollen. Hierfür weist das Tablar 61 bevorzugt Befestigungselemente, z.B. für einen Gefässständer, auf, um die Proben 69 bzw. Gefässe, insbesondere Mikrotiterplatten, während des Schüttelvorgangs relativ zum Tablar 61 ortsfest zu halten.

Das Tablar 61 ist über eine fest verbundene Tablarwelle 67 drehbar an dem Antriebselement 66 gelagert. Das Antriebselement 66 ist wiederum an dem Träger 62 drehbar befestigt, welcher an der Hauptantriebswelle 63 über das Lager 63a schwenkbar gelagert ist. Die Lagerung der Tablarwelle 67 in oder an dem Antriebselement 66 ist exzentrisch, die Drehachse der Tablarwelle 67 fällt also nicht mit der Drehachse des Antriebselements 66 zusammen. Durch diese Exzentrizität der Tablarwelle 67 entsteht bei Rotation des Antriebselements 66 eine Kreisbewegung, auf der das Tablar 61 umläuft, und somit das gewünschte Schütteln des Tablars 61 samt Proben 69.

Optional wird das Tablar 61 im eingeschwenkten Zustand von einem Gehäuse 64 umschlossen, welches wie oben beschrieben als Verspritzschutz und/oder zur Klimatisierung der Proben dient. Die Hauptantriebswelle 63 verläuft, insbesondere vertikal, also in Richtung der Schwerkraft, durch das Gehäuse 64 hindurch und ist an diesem frei drehbar gelagert. Weiterhin ist ein Motor 65 zum Antreiben der Hauptantriebswelle 63, bevorzugt aussen, am Gehäuse 64 befestigt.

Ausserdem kann das Gehäuse 64 (wie bereits in Bezug auf Fig. 1a beschrieben) Füsse 64b umfassen, die dazu eingerichtet sind, das Gewicht der Vorrichtung zu tragen. Im Allgemeinen können die Füsse auch für eine Befestigung an einer Unterlage, z.B. an einem Labortisch, eingerichtet sein.

Fig. 6b ist eine vergrösserte Ansicht von Ausschnitt C in Fig. 6a. Das Tablar 61, das mit Proben 69 z.B. in einer Mikrotiterplatte bestückt werden kann, ist über die Tablarwelle 67 drehbar an dem Antriebselement 66 gelagert. Das Antriebselement 66 umfasst bevorzugt eine Riemenscheibe, die über einen Riemen 68 von der Hauptantriebswelle angetrieben wird. Dazu ist an der Hauptantriebswelle eine zweite Riemenscheibe angebracht und der Riemen 68 ist über die beiden Riemenscheiben gespannt. Vorteilhafterweise ist das Tablar 61 auch an der zweiten Riemenscheibe in gleicher Weise exzentrisch gelagert wie an der ersten Riemenscheibe am Antriebselement 66. Dies stellt eine Verdrehsicherung für das Tablar 71 dar, da seine Bewegungsfreiheit damit auf eine kreisförmige Translation eingeschränkt wird. Ausserdem kann das Tablar zur Verdrehsicherung, wie oben beschrieben, zwei Tablarteile umfassen, die miteinander z.B. durch eine Linearführung verbunden sind.

Im Allgemeinen ist es (wie oben beschrieben) vorteilhaft, Unwuchten, die an rotierenden Bauteilen der Vorrichtung auftreten, auszugleichen. Dies betrifft neben der Hauptantriebswelle, vgl. oben den Abschnitt "Mehrere Tablare", vor allem auch das Antriebselement 66. Insbesondere bei hohen Schüttelfrequenzen und daher Drehzahlen, z.B. von über 1000 rpm, über 1500 rpm oder gar über 2000 rpm, wie sie die Vorrichtung erreichen kann, führt eine Unwucht sonst zu Vibrationen, zu erhöhtem Verschleiss der Lager und Lagerungen sowie zu übermässiger Lärmentwicklung.

Fig. 6b zeigt eine Anordnung eines Gegengewichts 66a an dem Antriebselement 66, welche besonders einfach und wirkungsvoll eine Unwucht ausgleicht, die durch die exzentrische Lagerung des Tablars 61 (mit Proben 69 und Tablarwelle 67) an dem Antriebselement 66 hervorgerufen wird. Dabei befindet sich der Schwerpunkt SP2 des Gegengewichts 66a in der gleichen, orthogonal zur Drehachse des Antriebselements 66 verlaufenden Ebene wie der Schwerpunkt SP1 des Tablars 61 samt der vorgesehenen Beladung mit Proben 69 und der Tablarwelle 67.

Im Allgemeinen gilt: Dadurch dass die beiden Schwerpunkte SP1 und SP2 in der gleichen Ebene orthogonal zur Drehachse des Antriebselements 66 liegen, ist der oben (zu Fig. 5a) definierte Achsabstand l=0. Die beiden bei einer Rotation um die Drehachse auftretenden Kräfte F1 und F2 greifen also im gleichen Punkt an der Drehachse an. Somit gibt es kein Deviationsmoment D = U*l = 0 und das Auftreten einer dynamischen Unwucht wird per Design verhindert. Wenn zusätzlich die Bedingung zum Ausgleich einer statischen Unwucht (wie oben zu Fig. 5a beschrieben) erfüllt ist, nämlich U1 = U2, wird auch diese vermieden.

Für das Ausgleichen der Unwucht in der Praxis gilt die obige Bedingung insbesondere als erfüllt, wenn die Unwuchten U1 und U2 von Tablar 61 samt Beladung 69 und Tablarwelle 67 einerseits und vom Gegengewicht 66a andererseits um höchstens 25% voneinander abweichen. In Bezug auf den Achsabstand l, also den Abstand der Schwerpunkte SP1 und SP2 in Richtung der Drehachse, gilt die obige Bedingung insbesondere als erfüllt, wenn der Achsabstand l höchstens 1 cm beträgt. Diese Toleranzen erlauben, dass die Unwucht auf das Antriebselement 66 in der Praxis auch bei kleineren Abweichungen, z.B. in Bezug auf Masse oder Massenverteilung, von der definierten Beladung ausreichend ausgeglichen wird.

Dies kann gemäss Fig. 6b so gelöst werden, dass das Tablar 61 eine Ausstülpung 61a nach oben umfasst, unter welcher sich zumindest ein Teil des Gegengewichts 66a befindet. Die Drehmomente, die bei Rotation des Antriebselements 66 um die Drehachse durch SP1 und SP2 ausgeübt werden, sollen sich im Allgemeinen gerade aufheben. Mit der gezeigten Anordnung lassen sich sowohl eine statische als auch eine dynamische Unwucht ausgleichen. Dies ermöglicht es, mit einem platzsparenden Aufbau hohe Schüttelfrequenzen von über 1000 rpm, insbesondere über 1500 rpm oder über 2000 rpm, zu erreichen. Gleichzeitig ermöglicht dies aufgrund der Vermeidung von erhöhtem Verschleiss eine lange Lebensdauer und einen Dauerbetrieb des Schüttlers.

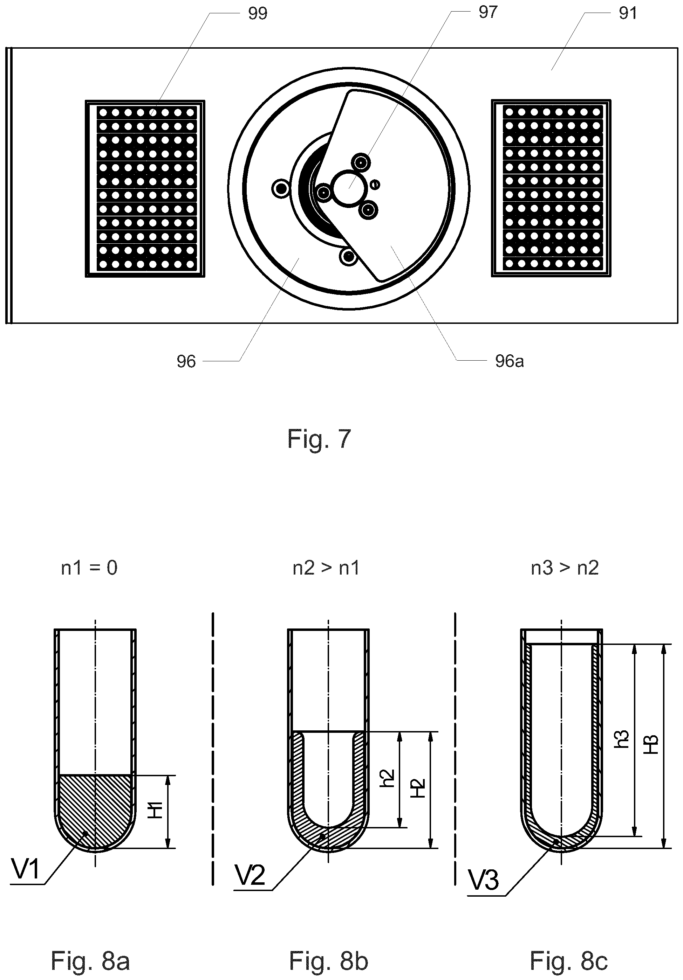

Fig. 7 zeigt eine Draufsicht von oben auf bzw. einen horizontalen Schnitt durch ein Tablar 91 mit Gegengewicht 96a. Auf dem Tablar 91 sind z.B. mittels Gefässhaltern zwei Mikrotiterplatten mit einer Vielzahl von Proben 99 angebracht. Wie oben im Zusammenhang mit Fig. 6b beschrieben, ist das Gegengewicht 96a an dem Antriebselement 96 angebracht, sodass besonders einfach und wirkungsvoll eine Unwucht ausgeglichen wird, die durch die exzentrische Lagerung des Tablars 91 (mit Proben 99) an dem Antriebselement 96 hervorgerufen wird. Wie gezeigt kann das Gegengewicht 96a z.B. mit Schrauben an dem Antriebselement 96 befestigt sein. Wiederum befindet sich der Schwerpunkt des Gegengewichts 96a in der gleichen, orthogonal zur Drehachse des Antriebselements 96 verlaufenden Ebene wie der Schwerpunkt des Tablars 91 samt der vorgesehenen Beladung mit Proben 99.

Vorteilhafterweise umfasst das Gegengewicht 96a eine Öffnung, durch welche die Tablarwelle 97 hindurch verläuft. Ausserdem kann das Gegengewicht 96a in der Draufsicht von oben vorteilhafterweise ähnlich einem Kreissektor geformt sein. Beide Ausgestaltungen ermöglichen ein möglichst grosses Volumen und damit eine möglichst grosse Masse des Gegengewichts 96a, wobei das Gegengewicht 96a dennoch mit dem Antriebselement 96 in der Ausstülpung des Tablars 91 umlaufen kann. Dadurch wird der für die Proben 99 auf dem Tablar 91 verfügbare Platz maximiert.

Figs. 8a, 8b und 8c illustrieren den Effekt verschiedener Schüttelfrequenzen n1, n2 bzw. n3 auf eine flüssige Probe, die sich in einem Gefäss, z.B. einem Reagenzglas oder einer Mikrotiterplatte, befindet. Das Probenvolumen V ist in jeder der Abbildungen gleich: V1 = V2 = V3.

In Fig. 8a ist die Probe in Ruhe, also Schüttelfrequenz n1 = 0. Die Probenflüssigkeit weist eine annähernd ebene und waagrechte Oberfläche auf. Die Probenflüssigkeit füllt das Gefäss dabei bis zu einer Höhe H1. Diese Höhe kann als Mass für die Diffusionsstrecke d1 genommen werden, welche Sauerstoff aus dem umgebenden Gas in die Probe hinein zurücklegen muss: d1 = H1.

In Fig. 8b wird die Probe mit einer Schüttelfrequenz n2 > 0 geschüttelt, z.B. mit n2 = 1000 rpm. Dabei wird die Flüssigkeit am Gefässrand nach oben gedrückt und es bildet sich ein Meniskus, also eine konkave Oberfläche der Probenflüssigkeit aus. Während die Oberfläche gegenüber der Situation in Fig. 8a zunimmt, nimmt die Diffusionsstrecke d2 = H2-h2 ab: d2 < d1. Beides führt dazu, dass Sauerstoff schneller in die Probe gelangt.

In Fig. 8c wird die Probe noch deutlich schneller geschüttelt, n3 > n2, z.B. mit n3 = 2000 rpm. Die Probenflüssigkeit wird weit am Gefässrand nach oben "gezogen". Die Oberfläche nimmt infolge des sich verstärkenden Meniskus weiter zu und die Diffusionsstrecke d3 = H3-h3 nimmt weiter ab: d3 < d2. Der Sauerstofftransport in die Probe ist also nochmals verbessert.

Damit ist einer der grossen Vorteile der beschriebenen Vorrichtung gezeigt, welche Schüttelfrequenzen von über 1000 rpm, insbesondere über 1500 rpm oder über 2000 rpm, erreichen kann: Der Sauerstofftransport aus der Gasphase in die Flüssigphase, also in die Probe hinein, wird stark erhöht. Insbesondere lassen sich so Zellen mit einem ähnlichen schnellen Wachstum kultivieren und ähnlich viel Biomasse produzieren wie bei einer Kultivierung in einem Bioreaktor. Mit einer solchen Vorrichtung können also bereits erste Tests bei der Zellkultivierung unter ähnlichen Bedingungen durchgeführt werden wie später im ausgereiften Prozess, insbesondere im Bioreaktor.

Ausserdem zeigen die Figs. 8a, 8b und 8c auf, dass bei höheren Schüttelfrequenzen höhere Gefässe verwendet werden können und entsprechend eine grössere Probenmenge ausreichend durchmischt werden kann. So kann das Probenvolumen mindestens verdoppelt werden, z.B. von 1 ml auf 2 ml pro Einzelprobe in einer Deep Well Platte, und die Proben dennoch ausreichend durchmischt und mit Sauerstoff versorgt werden. Dies macht die Zellkultivierung erheblich zeitsparender und produktiver.

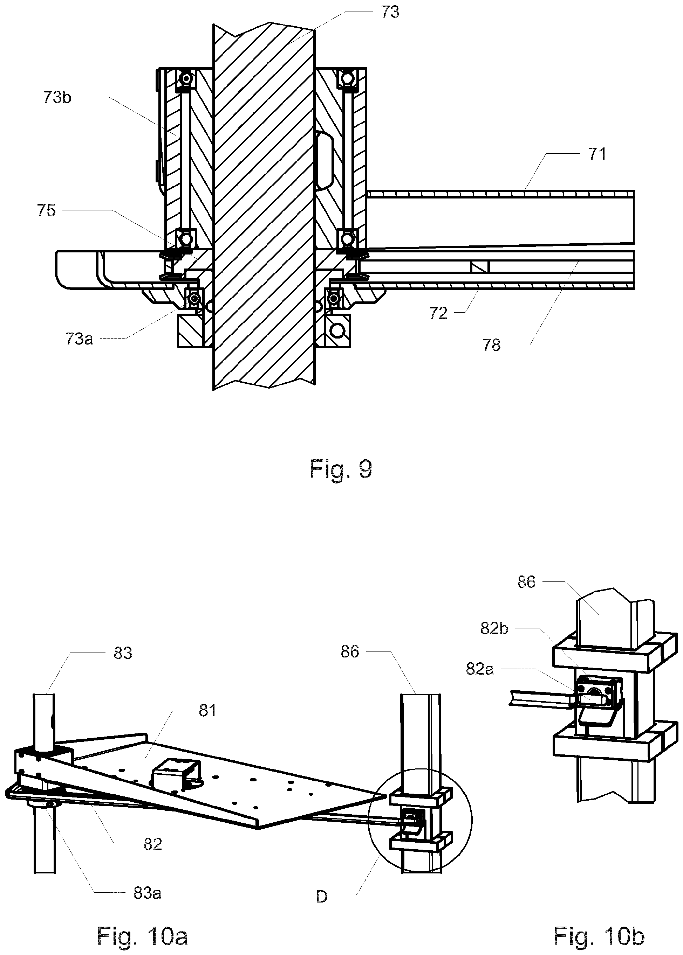

Fig. 9 zeigt, wie ein Tablar 71 über einen Träger 72 an einer Hauptantriebswelle 73 gelagert sein kann. Eine solche Lagerung ist z.B. kompatibel mit den Ausführungsformen der Figs. 1a/b, 3, 4 und 6a/b. Wie in Figs. 6a/b ist das Tablar 71 exzentrisch an einem Antriebselement (nicht gezeigt) mit erster Riemenscheibe gelagert. Das Antriebselement bzw. die erste Riemenscheibe ist an dem Träger 72 drehbar gelagert und eingerichtet, über den Riemen 78 angetrieben zu werden. Der Riemen 78 verläuft zusätzlich über die zweite Riemenscheibe 75, welche an der Hauptantriebswelle 73 befestigt ist und entsprechend vom Antrieb bzw. Motor über die Hauptantriebswelle 73 angetrieben wird.

Der Träger 72, welcher dazu eingerichtet ist, das Gewicht des Tablars 71 samt Beladung mit den Proben abzustützen, ist über ein Lager 73a, z.B. ein Kugellager, an der Hauptantriebswelle 73 gelagert. So kann der Träger 72 ortsfest bleiben, z.B. indem er über eine Verrastung wie in Figs. 10a/b arretiert wird, während die Hauptantriebswelle 73 rotiert. Bevorzugt befindet sich das Lager 73a unterhalb der zweiten Riemenscheibe 75.

Als Option kann der Träger 72 samt Tablar 71 alternativ oder zusätzlich auch oberhalb der zweiten Riemenscheibe 75 über ein zusätzliches Lager 73b an der Hauptantriebswelle 73 gelagert sein. Im Allgemeinen ist darauf zu achten, dass das Tablar für seine kreisförmige Translation, welche durch die exzentrische Lagerung verursacht wird, genügend Spielraum hat. Insbesondere muss eine Öffnung in dem Tablar 71, durch welche die Hauptantriebswelle 73 in einer bevorzugten Ausführungsform hindurchtritt, mindestens um die Exzentrizität der Lagerung grösser sein als der Durchmesser der Hauptantriebswelle 73 bzw. als das zweite Lager 73b, wenn vorhanden.

Mit einem Lager 73a bzw. 73b lassen sich auch mehrere Träger übereinander an einer Hauptantriebswelle 73 schwenkbar anbringen und gleichzeitig antreiben.

Figs. 10a und 10b illustrieren eine Möglichkeit, ein Tablar 81, das (wie z.B. im Zusammenhang mit Figs. 6a/b und 9 beschrieben) über ein Antriebselement (nicht sichtbar) an einem Träger 82 befestigt ist, für den Schüttelvorgang im Gehäuse bzw. an einer Stützstruktur 86 im Gehäuse zu arretieren, sodass es vorübergehend nicht um die Hauptantriebsachse schwenkbar ist. Die Stützstruktur 86 kann dabei Teil des Gehäuses oder ein separates Bauteil sein, das am Gehäuse befestigt ist. Für die Schwenkbarkeit des Tablars 81 ist der Träger 82 wiederum über ein Lager 83a an der Hauptantriebswelle 83 schwenkbar gelagert, siehe z.B. Fig. 9.

Fig. 10b zeigt den Ausschnitt D von Fig. 10a vergrössert. Dabei umfasst ein erstes Arretierelement 82a als Verrastung an oder nahe seinem Hauptantriebswellen-fernen Ende. Als Gegenstück zum ersten Arretierelement 82a ist an der Stützstruktur 86 ein zweites Arretierelement 82b angebracht. Das erste und zweite Arretierelement 82a und 82b sind insbesondere dazu eingerichtet, bei Kontakt eine lösbare Verbindung herzustellen. Dadurch kann der Träger 82 für den Schüttelvorgang mit der Stützstruktur 86 verbunden und insbesondere ein Schwenken des Trägers 82 um die Hauptantriebswelle 83 verhindert werden. Zusätzlich kann ein Teil des Gewichts von Träger 82 und Tablar 81 samt Proben von der Stützstruktur 86 getragen werden, was eine Belastung des Lagers 83a an der Hauptantriebsachse vermindert.

Im Allgemeinen umfasst die Verrastung z.B. mechanische oder magnetische Komponenten zum lösbaren Verbinden des Trägers 82 mit der Stützstruktur 86. Beispielsweise können die Arretierelemente 82a und 82b Magneten umfassen, welche dazu eingerichtet sind, durch ihre gegenseitige Anziehung den Träger 82 an der Stützstruktur 86 zu arretieren. Alternativ können die Arretierelemente 82a und 82b als Schnappverschluss oder als Klappverschluss ausgebildet sein, bei dem mechanisch eine lösbare Verbindung hergestellt wird.

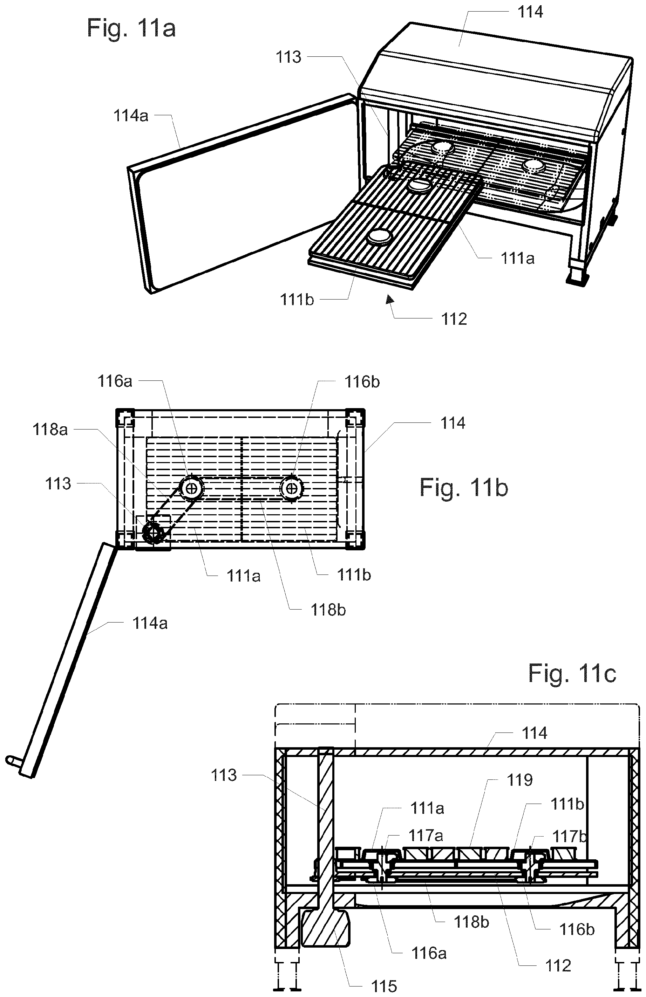

Figs. 11a, 11b und 11c illustrieren eine Ausführungsform des Schüttlers mit geteiltem Tablar, wodurch eine besonders einfache und zuverlässige Verdrehsicherung für das Tablar erreicht werden kann. Fig. 11a ist eine perspektivische Ansicht analog zu Fig. 1a; Fig. 11b ist ein schematischer Schnitt durch den Schüttler in der Ebene der Antriebselemente analog zu Fig. 1b; Fig. 11c ist ein schematischer vertikaler Schnitt analog zu Fig. 6a. Die zu den früheren Ausführungsformen beschriebenen Merkmale sind hier analog anwendbar.

Der Schüttler umfasst ein Gehäuse 114 mit einer Tür 114a, die zum Öffnen und Schliessen einer Gehäusevorderseite eingerichtet ist. In Figs. 11a und 11b ist die Tür 114a in geöffnetem Zustand dargestellt. Weiterhin umfasst der Schüttler eine Hauptantriebswelle 113, die von einem Motor 115 antreibbar ist. An der Hauptantriebswelle 113 ist ein Träger 112 drehbar gelagert, der z.B. mittels einer Verrastung (wie oben beschrieben) am Gehäsue eingerastet werden kann. Am Träger 112 sind wiederum ein erstes Antriebselement 116a und ein zweites Antriebselement 116b drehbar gelagert. Das erste Antriebselement 116a ist über einen ersten Riemen 118a an die Hauptantriebsachse 113 gekoppelt und wird von dieser angetrieben. Das zweite Antriebselement 116b ist über einen zweiten Riemen 118b an das erste Antriebselement 116a gekoppelt und wird damit ebenfalls angetrieben. Wichtig ist, dass die beiden Antriebselemente 116a und 116b synchron laufen. Daher wird zumindest für den zweiten Riemen 118b vorteilhafterweise ein Zahnriemen eingesetzt.

An oder in dem ersten Antriebselement 116a bzw. dem zweiten Antriebselement 116b ist eine erste Tablarwelle 117a bzw. eine zweite Tablarwelle 117b exzentrisch gelagert. An der ersten Tablarwelle 117a bzw. an der zweiten Tablarwelle 117b ist wiederum ein erster Tablarteil 111a bzw. ein zweiter Tablarteil 111b, insbesondere verdrehsicher, befestigt. Auf den Tablarteilen 111a und 111b können, wie früher beschrieben, Proben 119 angebracht werden, z.B. in Reagenzgläsern oder Mikrotiterplatten.

Eine besonders einfache und zuverlässige Verdrehsicherung für die beiden Tablarteile 111a und 111b kann nun über eine flexible Verbindung der beiden Tablarteile erreicht werden (in Figs. 11a-c nicht gezeigt). Vorteilhafterweise umfasst diese Verbindung eine Linearführung zwischen dem ersten Tablarteil 111a und dem zweiten Tablarteil 111b. Die Linearführung kann z.B. am einen Tablarteil fix befestigt sein, während sie eine Verschiebung des anderen Tablarteils entlang der Führung zulässt. Durch eine solche flexible Verbindung werden schädliche Kräfte auf die Lager der Tablarwellen und Antriebselemente, z.B. infolge von Wärmeausdehnung insbesondere des Trägers 112, vermieden.

Alternativ kann die Vorrichtung auch zwei Antriebselemente 116a und 116b wie in den Figs. 11a bis 11c gezeigt umfassen, die synchron angetrieben werden, ohne dass das Tablar in zwei Tablarteile 111a und 111b gezeigt ist. In diesem Fall ist das Tablar einstückig ausgebildet und (dennoch wie in Figs. 11a bis 11c gezeigt) über die erste Tablarwelle 117a und 117b an den Antriebselementen 116a und 116b gelagert. Auch in diesem Fall ist das Tablar gegen Rotation gesichert, sodass insbesondere keine zusätzlichen Führungselemente, z.B. Federn, zwischen Tablar und Gehäuse 114 erforderlich sind. Dies trägt wiederum zur Erreichung der erfindungsgemässen hohen Drehzahlen bei.
Während in der vorliegenden Anmeldung bevorzugte Ausführungen der Erfindung beschrieben sind, ist klar darauf hinzuweisen, dass die Erfindung nicht auf diese beschränkt ist und in auch anderer Weise innerhalb des Umfangs der folgenden Ansprüche ausgeführt werden kann.

## Patentansprüche

1. Vorrichtung zum Schütteln von Proben umfassend
- einen Träger (62),
- ein Antriebselement (66), das um eine Antriebsachse drehbar am Träger (62) gelagert und durch einen Antrieb (65) antreibbar ist,
- ein Tablar (61) eingerichtet zur Beladung mit den Proben (69),
- eine Tablarwelle (67), die mit dem Tablar (61) verbunden und exzentrisch an dem Antriebselement (66) gelagert ist,
- ein an dem Antriebselement (66) befestigtes Gegengewicht (66a), das angepasst ist, eine im Betrieb der Vorrichtung mit einer definierten Beladung des Tablars (61) auftretende Unwucht auszugleichen,
wobei sich ein Schwerpunkt des Gegengewichts (66a) bezogen auf die Antriebsachse des Antriebselements (66) gegenüber der Tablarwelle (67) befindet,
wobei sich der Schwerpunkt des Gegengewichts (66a) und ein Schwerpunkt des Tablars (61) samt Tablarwelle (67) und definierter Beladung in derselben, orthogonal zur Antriebsachse verlaufenden Ebene befinden,
wobei bei Rotation des Antriebselements (66) um die Antriebsachse ein erstes Drehmoment, das durch das Tablar (61) samt Tablarwelle (67) und definierter Beladung auf die Antriebsachse ausgeübt wird, betragsgleich und entgegengesetzt gerichtet ist zu einem zweiten Drehmoment, das durch das Gegengewicht (66a) auf die Antriebsachse ausgeübt wird,
wobei eine Schüttelfrequenz des Tablars (61) infolge des Antriebs (65) mindestens 1000 rpm beträgt,
insbesondere wobei das Gegengewicht (66a) so angepasst ist, dass es sowohl eine statische als auch eine dynamische Unwucht auf die Antriebsachse ausgleicht.

2. Vorrichtung nach Anspruch 1,
wobei das Gegengewicht (66a) das einzige Gegengewicht an dem Antriebselement (66) ist,
insbesondere wobei sich auf der dem Tablar (61) gegenüberliegenden Seite des Antriebselements (66) kein weiteres Gegengewicht befindet.

3. Vorrichtung nach einem der vorangehenden Ansprüche,
wobei die Tablarwelle (67) zentral an dem Tablar (61) befestigt ist.

4. Vorrichtung nach einem der vorangehenden Ansprüche,
wobei das Tablar (61) im Bereich der Tablarwelle (61) eine Ausstülpung (61a) aufweist,
wobei sich das Gegengewicht (66a) zumindest teilweise innerhalb der Ausstülpung (61a) befindet.

5. Vorrichtung nach einem der vorangehenden Ansprüche,
wobei das Gegengewicht (66a) eine Masse zwischen 0.1 und 1 kg aufweist,
insbesondere wobei das Gegengewicht (66a) aus Metall hergestellt ist.

6. Vorrichtung nach einem der vorangehenden Ansprüche,
wobei eine Exzentrizität der Lagerung der Tablarwelle (67) an dem Antriebselement (66) zwischen 0.5 und 3 mm beträgt, insbesondere zwischen 1 und 2 mm.

7. Vorrichtung nach einem der vorangehenden Ansprüche,
wobei eine Schüttelfrequenz des Tablars (61) infolge des Antriebs (65) mindestens 1500 rpm, insbesondere mindestens 2000 rpm oder mindestens 2500 rpm, beträgt.

8. Vorrichtung nach einem der vorangehenden Ansprüche,
wobei das Tablar (61) eine rechteckige Form aufweist,
insbesondere wobei das Tablar (61) eine Länge zwischen 50 und 100 cm aufweist, und/oder
insbesondere wobei das Tablar (61) eine Breite zwischen 30 und 70 cm aufweist.

9. Vorrichtung nach einem der vorangehenden Ansprüche,
wobei das Tablar (61) gegen Rotation gegenüber dem Träger (62) gesichert ist.

10. Vorrichtung nach Anspruch 9, weiterhin umfassend
- ein zweites Antriebselement, das um eine zweite Antriebsachse drehbar am Träger (62) gelagert und durch den Antrieb (65) antreibbar ist, insbesondere wobei das zweite Antriebselement über einen Zahnriemen mit dem Antriebselement (66) verbunden ist,
- eine zweite Tablarwelle, die mit dem Tablar (61) verbunden und exzentrisch an dem zweiten Antriebselement gelagert ist,
- ein an dem zweiten Antriebselement befestigtes zweites Gegengewicht, das angepasst ist, eine im Betrieb der Vorrichtung mit einer definierten Beladung des Tablars (61) auftretende Unwucht auszugleichen,
wobei sich ein Schwerpunkt des zweiten Gegengewichts bezogen auf die zweite Antriebsachse des zweiten Antriebselements gegenüber der zweiten Tablarwelle befindet,
wobei sich der Schwerpunkt des zweiten Gegengewichts und der Schwerpunkt des Tablars (61) samt zweiter Tablarwelle und definierter Beladung in derselben, orthogonal zur zweiten Antriebsachse verlaufenden Ebene befinden,
wobei bei Rotation des zweiten Antriebselements um die zweite Antriebsachse ein drittes Drehmoment, das durch das Tablar (61) samt zweiter Tablarwelle und definierter Beladung auf die zweite Antriebsachse ausgeübt wird, betragsgleich und entgegengesetzt gerichtet ist zu einem vierten Drehmoment, das durch das zweite Gegengewicht auf die zweite Antriebsachse ausgeübt wird.

11. Vorrichtung nach Anspruch 9,
wobei das Tablar einen ersten Tablarteil (111a) und einen zweiten Tablarteil (111b) umfasst,
wobei der erste Tablarteil (111a) und der zweite Tablarteil (111b) über eine erste Tablarwelle (117a) bzw. eine zweite Tablarwelle (117b), die mit dem ersten bzw. dem zweiten Tablarteil verbunden sind, exzentrisch an einem ersten Antriebselement (116a) bzw. einem zweiten Antriebselement (116b) gelagert sind,
wobei das erste Antriebselement (116a) und das zweite Antriebselement (116b) drehbar am Träger (112) gelagert und über die Hauptantriebswelle (113) antreibbar sind,
wobei der erste und der zweite Tablarteil durch eine flexible Verbindung, insbesondere durch eine Linearführung, miteinander verbunden sind.

12. Vorrichtung nach einem der vorangehenden Ansprüche,
wobei das Antriebselement (66) eine Riemenscheibe zum Antreiben über einen Riemen (68) aufweist.

13. Vorrichtung nach einem der vorangehenden Ansprüche, weiterhin umfassend
- ein öffenbares Gehäuse (64), insbesondere eingerichtet zur Klimatisierung eines Innenraums des Gehäuses (64),
wobei sich das Tablar (61), die Tablarwelle (67), das Antriebselement (66) und der Träger (62) im Innenraum befinden.

14. Vorrichtung nach einem der vorangehenden Ansprüche, weiterhin umfassend
- mindestens ein weiteres Tablar (41, 61), insbesondere mindestens fünf weitere Tablare, samt weiterer Tablarwelle, weiterem Antriebselement, weiterem Gegengewicht und weiterem Träger im Innenraum des Gehäuses (44),
wobei das Antriebselement (66) und das mindestens eine weitere Antriebselement über eine Hauptantriebswelle (43, 63) antreibbar sind.

15. Vorrichtung nach einem der Ansprüche 13 oder 14,
wobei der Antrieb (65) einen Motor zum Antreiben des Antriebselements (66) und, wenn vorhanden, des mindestens einen weiteren Antriebselements, insbesondere über die Hauptantriebswelle (63), umfasst,
wobei der Motor ausserhalb des Gehäuses (64) angebracht ist,
insbesondere wobei der Innenraum des Gehäuses (64) thermisch von dem Motor entkoppelt ist.
